# EUROPEAN PATENT APPLICATION

(11) **EP 2 628 592 A1**
(43) Date of publication of application: **21.08.2013**
(21) Application number: 13155349.7
(22) Date of filing: 15.02.2013
(51) Int. Cl.: B32B 3/28, B32B 27/12, B32B 9/02, B32B 3/26, B32B 9/04, A61F 2/00

(54) **Implantable devices including a mesh and a perforated film**

(30) Priority: 16.02.2012 US 201261599526 P; 24.01.2013 US 201313749090
(71) Applicant: Covidien LP, Mansfield, MA 02048 (US)
(72) Inventor: Stopek, Joshua, Louis Park, MN Minnesota 55416 (US); Elachchabi, Amin, Hamden, CT Connecticut 06518 (US); Broom, Daniel, Branford, CT Connecticut 06405 (US)
(74) Representative: Soames, Candida Jane

(57) **Abstract**

The present disclosure relates to implantable medical devices which include at least one mesh and at least one perforated film positioned on the mesh, wherein a portion of the film is positionable away the mesh to allow direct treatment of the contacting mesh tissue and the distal wound or incision site.

## Description

### CROSS-REFERENCE TO RELATED APPLICATIONS

This application claims the benefit of and priority to U.S. Provisional Patent Application No. 61/599,526, filed February 16, 2012, the entire disclosure of which is incorporated by reference herein.

### BACKGROUND

### Technical Field

The present disclosure relates generally to implantable medical devices, and more particularly, to implantable medical devices which include at least one mesh and a perforated film positioned on at least a portion of the mesh.

### Background of Related Art

Surgical meshes may be used during both laparoscopic and open surgery for repair of many types of defects and injuries. For example, surgical meshes are commonly used in the repair of hernias. The meshes may be used to provide support to surrounding tissue.

During hernia repair, a mesh may be placed over the entirety of damaged tissue and some of the healthy tissue surrounding the defect. The mesh can be held in place by a fixation device that attaches the mesh to the surrounding tissue. The mesh may further include an additional layer such as a film, for sustained delivery of analgesic agents to the vicinity of the mesh implant for reduction of acute post-operative pain.

Unlike prior art films which may be completely affixed to the mesh, the perforated portions of the present implant provide the implant with the ability to manipulate the film without manipulating the mesh and vice-versa. Integration of films to accommodate unique patient/anatomical features while maintaining the integrity of the film/mesh attachment is desired.

### SUMMARY

Accordingly, the present disclosure relates to implantable medical devices which include a surgical mesh and a perforated film positioned on at least a portion of the mesh. The mesh may generally be a textile or fabric created to promote tissue ingrowth and/or support injured tissue. The film may generally be polymeric in nature and may be intended to further enhance the ingrowth of tissue into the implant, prevent adhesions of surrounding tissue, deliver therapeutic agents and/or simply provide additional support to the implant. In certain embodiments, at least a portion of the film is fixedly attached to the mesh.

In embodiments, the film may include at least one perforated portion configured and dimensioned to be positionable away from the mesh to allow direct treatment of patient tissue. In certain embodiments, the film includes a plurality of perforations configured and dimensioned such that at least one portion of the film is fixedly attached to the mesh and at least one portion of the film is positionable away from the mesh.

The implantable medical device may further include at least one therapeutic agent. In embodiments, the film may be a single layer. In other embodiments, the film may include multiple polymeric layers.

Methods of delivery of a therapeutic agent are also provided which includes implanting a medical device including a therapeutic agent, mesh, and a film positioned on at least a portion of the mesh, wherein the film includes at least one perforation; trimming at least one perforated portion of the film to a predetermined configuration; and positioning the trimmed perforated portion away from the mesh and onto a wound site. In certain embodiments, the methods further include rolling the medical device; inserting the rolled medical device through an instrument having an elongated tubular member; and positioning the medical device at or near the wound site.

### BRIEF DESCRIPTION OF THE DRAWINGS

The foregoing objects and advantages of the disclosure will become more apparent from the reading of the following description in connection with the accompanying drawings, in which:

FIG. 1A is a top view of an implantable medical device according to one embodiment described in the present disclosure;

FIGS. 1B and 1C are a perspective view and a side view, respectively, of the implantable medical device of FIG. 1A with a film positionable away from a mesh according to one embodiment described in the present disclosure;

FIG. 2A is a top view of an implantable medical device according to one embodiment described in the present disclosure;

FIG. 2B is a top view of the implantable medical device of FIG. 2A with at least one portion of a perforated film completely removed from a mesh according to another embodiment described in the present disclosure;

FIGS. 2C and 2D are a top view and a side view, respectively, of the implantable medical device of FIG. 2A with at least one portion of a perforated film positionable away from a mesh according to one embodiment described in the present disclosure;

FIG. 3A is a top view of an implantable medical device according to one embodiment described in the present disclosure;

FIG. 3B is a perspective view of the implantable medical device of FIG. 3A illustrating a tent-like configuration of a film on a mesh;

FIGS. 3C and 3D are perspective views of the implantable medical device of FIG. 3A with at least two perforated film portions positionable away from a mesh;

FIG. 4 is a perspective view of an implantable medical device in a rolled configuration for implantation into a wound site according to one embodiment described in the present disclosure;

FIG. 5 is a diagram showing the weave of three sheets forming a medical device according to one embodiment described in the present disclosure; and

FIG. 6 is a diagrammatic side view of a device permitting the formation of spiked naps on the medical device of FIG. 5 according to another embodiment described in the present disclosure.

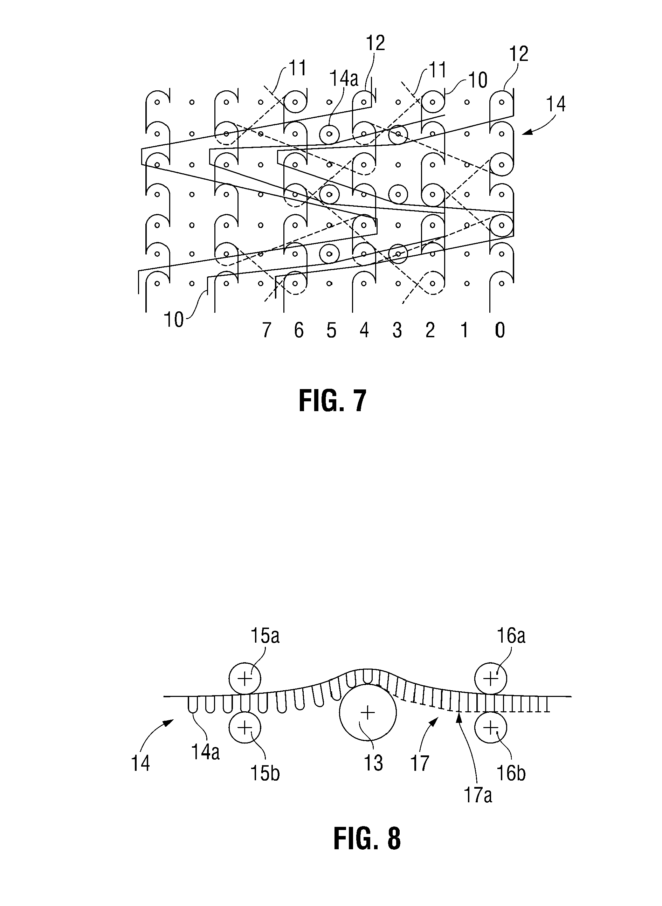

FIG. 7 is a diagram showing the weave of three sheets forming a medical device according to one embodiment described in the present disclosure; and

FIG. 8 is a diagrammatic side view of a device permitting the formation of spiked naps on the medical device of FIG. 7 according to another embodiment described in the present disclosure.

### DETAILED DESCRIPTION

The present disclosure relates to implantable medical devices which include a surgical mesh and a perforated film. By perforated, the film may include a series of small holes, fenestrations, punctures, or slits which are arranged in an organized manner. The perforations are configured and dimensioned to allow a user to separate a first portion of the film from a second portion of the film in situ or prior to implantation. The perforated film can be easily separated into sections and/or torn easily along the perforated line. In embodiments, the perforated film may be positioned in a variety of ways on at least a portion of the mesh. In embodiments, the film may be further trimmed and/or positioned away from the mesh while still maintaining some point of attachment to the mesh. In embodiments, the film includes at least one perforation configured and dimensioned such that at least one perforated portion of the film may be at least partially separated from the mesh.

In embodiments, a film may include a plurality of perforations configured and dimensioned such that at least one portion of the film is fixedly attached to the mesh and at least one perforated portion of the film is positionable away from the mesh.

In some embodiments, the film includes at least one perforated portion configured and dimensioned to be separated from the mesh to allow the perforated portion to protrude from a surface of the mesh. In such a position, the portion of the film which is separated from the mesh can be positioned into the wound tissue. The perforated film may be trimmed in situ or prior to implantation to a predetermined customized shape or orientation. In embodiments, the film may include graduated markings so that the defined portions of the film are easily identifiable for trimming.

In embodiments, at least one portion of the film is attached to the mesh. In embodiments, the film may be attached to the mesh at a peripheral edge(s) of the film. In other embodiments, the film may be attached to the mesh at an interior edge(s) of the film, such as in embodiments wherein the film includes an aperture which creates at least one interior edge. In still other embodiments, the film may be attached to the mesh near the interior of the mesh (towards the center). In yet other embodiments, the film may be attached to the mesh on both interior and exterior portions so as to form a tent-like attachment configuration wherein the laminar face of the film is free of the mesh.

In embodiments, the perforated film may include pre-cut holes or notches in the face of the film for easy manipulation and/or trimming of perforated portions of the film providing for use of either one or multiple individual film regions on the mesh. In alternative embodiments, the film may be pierced with a surgical tool such as a scalpel or grasper for easy manipulation of perforated portions of the films.

By implantable, the medical devices described herein may be positioned, for any duration of time, at a location within a body, such as within a portion of the abdominal cavity. Furthermore, the terms "implantation" and "implanted" refer to the positioning, for any duration of time, of a medical device at a location within a body, such as within a portion of the abdominal cavity.

The implantable medical devices described herein include at least one surgical mesh. The surgical mesh described herein may include porous fabrics made from intertwined filaments. The filaments may be monofilaments or multi-filaments and, in embodiments, a plurality of multi-filaments may be combined to form yarns. The filaments may extend horizontally and vertically in a manner which produces sections where the filaments cross-over one another creating points of common intersection. The surgical mesh may be woven, non-woven, knitted or braided. In some embodiments, the filaments may form two-dimensional or three-dimensional meshes. Some examples of two-dimensional and/or three-dimensional mesh substrates may be found in U.S. Patent No. 7,021,086, U.S. Patent No. 6,596,002, U.S. Patent No. 7,331,199, the entire contents of which are incorporated by reference herein.

Suitable meshes for use in the present disclosure include, for example, a collagen composite mesh such as PARIETEX™ Composite Mesh (commercially available from Tyco Healthcare Group LP, d/b/a Covidien). PARIETEX™ Composite Mesh is a 3-dimensional polyester weave with a resorbable collagen film bonded on one side. Another suitable mesh includes Parietex Progrip™ self-fixating mesh (also commercially available from Covidien). Parietex Progrip ™ is a polyester mesh which includes poly lactic acid (PLA) grip members. Other suitable meshes include those sold under the names PARIETENE®, PARIETEX™, SURGIPRO™ (all commercially available from Covidien); PROLENE™ (commercially available from Ethicon, Inc.); MARLEX®, DULEX®, 3D MAX® mesh, PERFIX® plug, VENTRALEX®, and KUGEL® patch (all commercially available from C.R. Bard, Inc.); PROLITE ™, PROLITE ULTRA™ (all commercially available from Atrium Medical); COMPOSIX®, SEPRAMESH®, and VISILEX® (all commercially available from Davol, Inc.); and DUALMESH®, MYCROMESH®, and INFINIT® mesh (all commercially available from W.L. Gore). Additionally, meshes within the scope and context of this disclosure may include biologic materials such as allografts (i.e., AlloDerm® Regenerative Tissue Matrix from Lifecell), autografts, and xenografts (i.e., PERMACOL™, from Covidien). In alternate embodiments, processed/purified tissues may also be employed.

In certain embodiments, Parietex™ Composite Mesh or Parietex™ Pro-grip may be utilized in accordance with the present invention.

In certain embodiments, the medical device may be a surgical mesh knitted on a warp knitting machine, of the tricot or Raschel type, with at least three sheets or warps of yarn and as many guide bars.

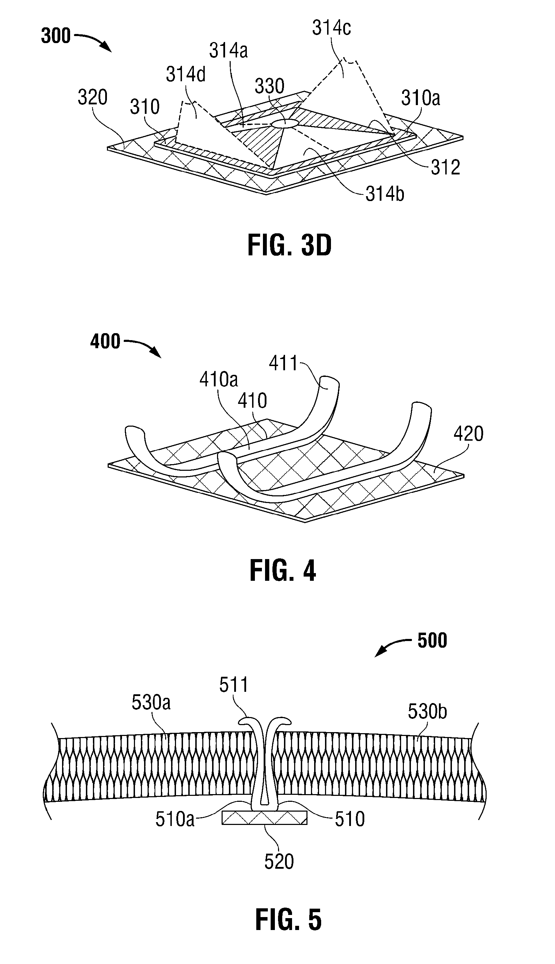

A rear bar is threaded, one guide full and one guide empty, with first mono- or multi-filaments 10 of a biocompatible polymer as represented as a solid line in FIG. 5. An intermediate bar is threaded, one guide full, three guides empty, with second mono- or multi-filaments 11 of a biocompatible polymer as represented as a broken line in FIG. 5. The intermediate bar works in such a way as to obtain a zigzag openwork pattern between the columns of meshes. Finally, a front bar is threaded, one guide full, one guide empty, and works in a chain stitch with third mono- or multi-filaments 12 a biocompatible polymer as represented by a thin line in FIG. 5. The third filament 12, i.e., a chain stitch, imprisons first filament 10 and maintains the length of the mesh while contributing to the formation of the mesh with the intermediate sheet formed by the second filament 11. The different filaments may form yarns and may be worked according to the following chart:

| **Warp →** | **Rear bar I** | **Intermediate bar II** | **Front bar III** |
|---|---|---|---|
| **Raschel →** | **Front bar I** | **Intermediate bar II** | **Rear bar III** |
| | 7 | 3 | 1 |
| | 7 | 2 | 0 |
| | — | — | — |
| | 3 | 4 | 0 |
| | 4 | 5 | 1 |
| | — | — | — |
| | 0 | 1 | |
| | 0 | 0 | |
| | — | — | |
| | 4 | 2 | |
| | 3 | 3 | |
| | | — | |
| | | 1 | |
| | | 0 | |
| | | — | |
| | | 4 | |
| | | 5 | |

The rear bar places the first filament or yarn in partial weft under the chain stitch and "thrown" onto the needle not forming a chain stitch. For this reason, at the next row, the needle not forming a chain stitch not being supplied permits escape of the filament which forms a loop 14a projecting from the front face of the mesh.

The threading-one guide full, three guides empty-in the intermediate bar, associated with the displacement, makes it possible to form a light ground texture, stable in width, and open-worked to permit good tissue integration.

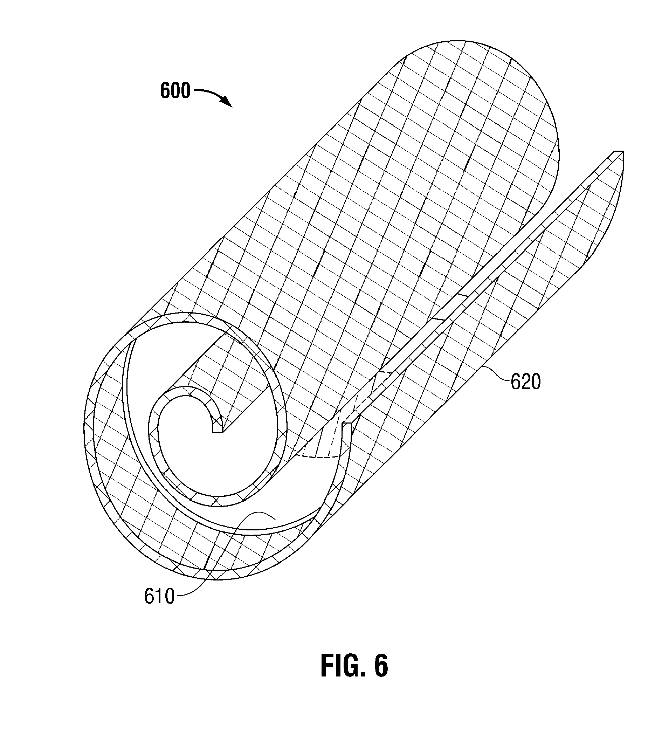

The mesh 14 thus obtained may be provided with loops 14a (FIG. 6) which may be perpendicular to one of the mesh surfaces. Loops 14a may also include a rigidity and hold at a right angle which may be obtained by the rigidity or nerve of the filaments employed. This rigidity may be necessary for the subsequent formation of grip members which ensure a grip function to at least a portion of the implantable medical device.

On leaving the loom, mesh 14 may be subjected to a thermosetting operation which stabilizes the mesh length and width. The mesh may then be subjected to a phase of formation of the grip members consisting, as is shown in FIG. 6, in passing the mesh over a cylinder 13 containing an electrical heating resistor. Mesh 14 is pressed flat on cylinder 13 by two pairs of rollers, upstream 15a, 15b and downstream 16a, 16b, respectively, which are vertically displaceable for controlling this pressing force.

This control as well as that of the temperature of the resistor placed in cylinder 13 and of the speed of movement of mesh 14 across cylinder 13 make it possible to melt the head of each of loops 14a so that each loop 14a forms two grip members 17.

Each grip member 17 thus may have a substantially rectilinear body protruding perpendicularly with respect to mesh 14 and, at the free end of this body, a head 17a of greater width than that of the body. Head 17a has a generally spheroidal shape or a mushroom shape. Grip member 17 gives mesh 14 the ability to attach to tissue when implanted. In addition, grip members 17 may attach to other portions of mesh 14 when folded or rolled. The grip members may be positioned along any portion of the mesh and in any quantity and/or configuration. For example, in some embodiments, the grip members may be positioned on the same portion of the mesh as the film. In other embodiments, the grip members may be positioned on a different portion of the mesh which does not include the film.

Any biocompatible material may be used to form the mesh described herein. For example, the mesh may be made from non-bioabsorbable materials, such as polypropylene, polyethylene terephthalate, polytetrafluoroethylene, and the like. In other examples, the mesh may be made from bioabsorbable materials, such as polylactide, polyglycolide, polycaprolactone, polydioxanone, polysaccharides and the like. In embodiments, the mesh may be made from a combination of absorbable and non-bioabsorbable materials.

The medical devices described herein may be formed using any method within the purview of those skilled in the art. Some non-limiting examples include, weaving, knitting, braiding, crocheting, extruding, spraying, casting, molding, and combinations thereof. In embodiments, the medical device may include a two or three dimensional surgical mesh which is woven, knitted, braided, or crocheted.

The medical devices described herein may include a polymeric film layer which may be made from any biocompatible material. The biocompatible material may be a homopolymer or a copolymer, including random copolymer, block copolymer, or graft copolymer. The biocompatible material may be a linear polymer, a branched polymer, or a dendrimer. The biocompatible material may be of natural or synthetic origin. The biocompatible material may be bioabsorbable or non-bioabsorbable.

Some additional non-limiting examples of bioabsorbable materials used to form the medical device include polymers selected from the group consisting of aliphatic polyesters; polyamides; polyamines; polyalkylene oxalates; poly(anhydrides); polyamidoesters; copoly(ether-esters); poly(carbonates) including tyrosine derived carbonates; poly(hydroxyalkanoates) such as poly(hydroxybutyric acid), poly(hydroxyvaleric acid), and poly(hydroxybutyrate); polyimide carbonates; poly(imino carbonates) such as such as poly (bisphenol A-iminocarbonate and the like); polyorthoesters; polyoxaesters including those containing amine groups; polyphosphazenes; poly (propylene fumarates); polyurethanes; polymer drugs such as polydiflunisol, polyaspirin, and protein therapeutics; biologically modified (e.g., protein, peptide) bioabsorbable polymers; and copolymers, block copolymers, homopolymers, blends, and combinations thereof.

More specifically, aliphatic polyesters include, but are not limited to, homopolymers and copolymers of lactide (including lactic acid, D-,L- and meso lactide); glycolide (including glycolic acid); epsilon-caprolactone, p-dioxanone (1,4-dioxan-2-one); trimethylene carbonate (1,3-dioxan-2-one); alkyl derivatives of trimethylene carbonate; Δ-valerolactone; β-butyrolactone; γ-butyrolactone; ε-decalactone; hydroxybutyrate; hydroxyvalerate; 1,4-dioxepan-2-one (including its dimer 1,5,8,12-tetraoxacyclotetradecane-7,14-dione); 1,5-dioxepan-2-one; 6,6-dimethyl- 1,4-dioxan-2-one; 2,5-diketomorpholine; pivalolactone; α, α diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and polymer blends and copolymers thereof.

Other suitable bioabsorbable materials may include but are not limited to poly(amino acids) including proteins such as collagen (I, II and III), elastin, fibrin, fibrinogen, silk, and albumin; peptides including sequences for laminin and fibronectin (RGD); polysaccharides such as hyaluronic acid (HA), dextran, alginate, chitin, chitosan, and cellulose; glycosaminoglycan; mucilage, pectin; and combinations thereof.

The term "collagen" is meant to include any type of collagen, whether natural or synthetic, of human or animal origin, such as, for example, enriched human collagen of type I, human collagen of type III, also enriched, human collagen of type I+III or of type IV or other collagens such as animal collagen of type I or of type I+III. The collagen may be oxidized or non-oxidized. In certain embodiments, the collagen may be oxidized without crosslinking. For example, native collagen may be dipped in an acid solution and/or washed, to eliminate the telopeptides, notably by pepsin digestion.

The collagen may also be modified by oxidative cleavage. For this purpose periodic acid or one of its salts can be used, applying the technique described by M. TARDY et al. (FR-A-2 601 371 and U.S. Pat. No. 4,931,546, the entire contents of which are herby incorporated by reference).

It is recalled briefly that this technique consists of mixing the collagen in acid solution with a solution of periodic acid or one of its salts at a concentration of between about 1 and about 10⁻⁵ M, in embodiments between about 5x10⁻³ and about 10⁻¹ M, at a temperature of between about 10°C and about 25°C for about 10 minutes to about 72 hours.

This process breaks down some of the collagen's components, these being hydroxylysine and the sugars, thus creating reactive sites without causing crosslinking.

The oxidative cleavage of collagen allows moderate cross-linking later in the collagenic material but does not exclude the possibility of providing this function by other means of moderate cross-linking, for example by beta or gamma irradiation, or other agents of moderate cross-linking, for example chemical reagents at suitably low and non-toxic doses.

For some applications, the polymer film layers described herein may include collagen which is not oxidized or a mixture in any proportions of non-oxidized and oxidized collagens. For example, in some embodiments, the film may include a film of oxidized collagen and non-oxidized collagen in equal parts. In other embodiments, the oxidized collagen may represent from about 25-75% of the film layer and the non-oxidized collagen may represent from about 25-75% of the film layer.

Particular examples of implantable medical devices disclosed herein include a polypropylene, polyethylene terephthalate, or polybutester mesh in combination with films comprising materials such as aliphatic polyesters (e.g., PGA, PLA, PLGA), polyorthoesters, hydroxybutyrates, collagens, hyaluronic acids, cellulose (e.g., carboxymethyl celluloses), vinyl polymers, non-degradable polyesters, and combinations thereof.

Both the mesh and/or the film may further consist of at least one optional ingredient. Some examples of suitable optional ingredients include emulsifiers, viscosity enhancers, dyes, pigments, fragrances, pH modifiers, wetting agents, plasticizers, antioxidants, and the like. The optional ingredients may represent up to about 10% of the mesh and/or film by weight.

In embodiments, the perforated film may include at least two sets of perforations creating at least three distinct sections of the film. In some embodiments, at least a portion of the middle film section is fixedly attached to the mesh and the two or more outer sections are configured and dimensioned to be positionable away from the mesh. In embodiments, the perforations do not extend completely across the entire length of the film allowing for two opposite ends of a film section, i.e. strips or fingers, to extend upward in a substantially perpendicular configuration to allow the strip or finger of film to penetrate into the wound or tissue while the middle film section stays fixed to the mesh. Further, it is envisioned that the films disclosed herein may include a single or multiple layers.

In embodiments, the film may include a plurality of perforations extending outward from a central region or area of the film forming, a plurality of perforated triangle-like portions. In some embodiments, the film may include a pre-cut hole or notch, significantly larger that the perforations, and a plurality of perforations may extend from the pre-cut hole or notch to an outer edge of the film to allow for easy manipulation of the perforated portions. In alternative embodiments, the surgeon may pierce the perforated film along the plurality of perforations with a scalpel or grasper to allow for easy manipulation of the perforated portions. In embodiments, the perforated film may be fixedly attached to the mesh on the exterior portions or edge of the film. In this way, outer perforated portions may be extended or raised away from the mesh by manipulation of the perforated portions via a precut hole which forms a "tent-like" arrangement to allow a portion of the film to penetrate into the wound or tissue and away from a surface of the mesh.

In embodiments, the film may extend beyond the mesh wherein interior portions of the film are fixedly attached to the mesh and outer portions not in contact with the mesh are trimmable or extendable to allow for direct treatment of a wound site. In other embodiments, the film may include a predetermined shape and configuration such that the film does not extend beyond the mesh and only a portion of the film is fixedly attached to the mesh such that outer fingers or strips of the film are extendable or raised into a perpendicular configuration for direct treatment of wound area or incision space.

The film and/or mesh may be pre-formed or cut into any suitable shape, such as for example, round, square, star shaped, octagonal, rectangular, polygonal, triangle, u-shaped, and oval. In embodiments, the film may be pre-formed or cut into rectangular strips.

In embodiments, perforations may be formed by any means known in the art such as for example, by pins, needles, dies, punches, blades, molds, lasers, ultrasonics, and combinations thereof.

The films described herein may be formed by any suitable method known to those skilled in the art. In certain embodiments, a solution may be formed which includes the suitable polymeric material and any optional ingredients. The solution may be cast bulk sheet stock, spray coated using an ultrasonic sprayer, extruded, molded and the like, to form the films described herein. Suitable solvents for making polymer solutions include, without limitation, methylene chloride, chloroform, N-methylpyrrolidone, tetrahydrofuran, dimethylformamide, methanol, ethanol, hexanes, acetone and combinations thereof.

In some embodiments, the film may be cast directly on a portion of the mesh surface. In other embodiments, the film may be spray coated directly on a portion of the mesh. In still other embodiments, the film may be formed before being attached to the mesh.

In yet another embodiment, the film may be perforated before being combined with the mesh. In yet another embodiment, the film may be perforated after being combined with the mesh. In yet still another embodiment, the polymeric film layer may be formed using an ultrasonic spraying nozzle onto an inert substrate.

More specifically, the film may be created using a spraying technique, such as ultrasonic spraying. For example, the films described herein may be fabricated by passing one or more solutions containing the polymer materials suitable for forming the film and optionally one or more therapeutic agents, through an ultrasonic spray nozzle to form droplets. The droplets may be mixed while falling towards or being deposited onto an inert substrate, such as silicone sheet, and/or a portion of the mesh to form a film. In some embodiments, prior to spraying the film, an inert substrate may be positioned on the portion of the mesh preventing film attachment. Thus, upon formation of the film, the film may adhere to the portions of the mesh which are not covered by the inert substrate and the film will not fixedly attach to the portions of the mesh which are covered by the inert substrate. The inert substrate may be removed prior to implantation.

Film may be attached to mesh utilizing methods including, but not limited to solvent welding, adhesives, films casting, and the like. Alternatively, the mesh and film may be sewn together, using, for example, a suture.

In certain embodiments, the implantable medical device includes a therapeutic agent. The term "therapeutic agent", as used herein, is used in its broadest sense and includes any substance or mixture of substances that provides a beneficial, therapeutic, pharmacological, and/or prophylactic effect. The agent may be a drug which provides a pharmacological effect.

The term "drug" is meant to include any agent capable of rendering a therapeutic affect, such as, anti-adhesives, antimicrobials, analgesics, antipyretics, anesthetics (e.g. local and systemic), antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, platelet activating drugs, clotting factors, and enzymes. It is also intended that combinations of agents may be used.

Other therapeutic agents, which may be included as a drug include: anti-fertility agents; parasympathomimetic agents; psychotherapeutic agents; tranquilizers; decongestants; sedative hypnotics; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents, such as coronary vasodilators and nitroglycerin; alkaloids; analgesics; narcotics such as codeine, dihydrocodeinone, meperidine, morphine and the like; non-narcotics, such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents, such as hormonal agents, hydrocortisone, prednisolone, prednisone, non-hormonal agents, allopurinol, indomethacin, phenylbutazone and the like; prostaglandins and cytotoxic drugs; chemotherapeutics; estrogens; antibacterials; antibiotics; anti-fungals; anti-virals; anticoagulants; anticonvulsants; antidepressants; and immunological agents.

Other examples of suitable agents, which may be included in the medical devise described herein include, for example, viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (e.g., IL-2, IL-3, IL-4, IL-6); interferons (e.g., β-IFN, α-IFN and γ-IFN); erythropoietin; nucleases; tumor necrosis factor; colony stimulating factors (e.g., GCSF, GM-CSF, MCSF); insulin; anti-tumor agents and tumor suppressors; blood proteins such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins; TGF-B; protein inhibitors; protein antagonists; protein agonists; nucleic acids such as antisense molecules, DNA, RNA, and RNAi; oligonucleotides; polynucleotides; and ribozymes.

Some specific non-limiting examples of water-soluble drugs that may be used in the present medical devices include, lidocaine, bupivicaine, tetracaine, procaine, dibucaine, sirolimus, taxol, chlorhexidine, polyhexamethylene, thiamylal sodium, thiopental sodium, ketamine, flurazepam, amobarbital sodium, phenobarbital, bromovalerylurea, chloral hydrate, phenytoin, ethotoin, trimethadione, primidone, ethosuximide, carbamazepine, valproate, acetaminophen, phenacetin, aspirin, sodium salicylate, aminopyrine, antipyrine, sulpyrine, mepirizole, tiaramide, perixazole, diclofenac, anfenac, buprenorphine, butorphanol, eptazocine, dimenhydrinate, difenidol, dl-isoprenaline, chlorpromazine, levomepromazine, thioridazine, fluphenazine, thiothixene, flupenthixol, floropipamide, moperone, carpipramine, clocapramine, imipramine, desipramine, maprotiline, chlordiazepoxide, clorazepate, meprobamate, hydroxyzine, saflazine, ethyl aminobenzoate, chlorphenesin carbamate, methocarbamol, acetylcholine, neostigmine, atropine, scopolamine, papaverine, biperiden, trihexyphenidyl, amantadine, piroheptine, profenamine, levodopa, mazaticol, diphenhydramine, carbinoxamine, chlorpheniramine, clemastine, aminophylline, choline, theophylline, caffeine, sodium benzoate, isoproterenol, dopamine, dobutamine, propranolol, alprenolol, bupranolol, timolol, metoprolol, procainamide, quinidine, ajmaline, verapamil, aprindine, hydrochlorothiazide, acetazolamide, isosorbide, ethacrynic acid, captopril, enalapril, delapril, alacepril, hydralazine, hexamethonium, clonidine, bunitrolol, guanethidine, bethanidine, phenylephrine, methoxamine, diltiazem, nicorandil, nicametate, nicotinic-alcohol tartrate, tolazoline, nicardipine, ifenprodil, piperidinocarbamate, cinepazide, thiapride, dimorpholamine, levallorphan, naloxone, hydrocortisone, dexamethasone, prednisolone, norethisterone, clomiphene, tetracycline, methyl salicylate, isothipendyl, crotamiton, salicylic acid, nystatin, econazole, cloconazole, vitamin B₁ , cycothiamine, vitamin B₂, vitamin B₃, vitamin B₅, vitamin B₆, vitamin B₇, vitamin B₉, vitamin B₁₂, vitamin C, nicotinic acid, folic acid, nicotinamide, calcium pantothenate, pantothenol, panthetin, biotin, ascorbic acid, tranexamic acid, ethamsylate, protamine, colchicine, allopurinol, tolazamide, glymidine, glybuzole, metoformin, buformin, orotic acid, azathioprine, lactulose, nitrogen mustard, cyclophophamide, thio-TEPA, nimustine, thioinosine, fluorouracil, tegafur, vinblastine, vincristine, vindesine, mitomycin C, daunorubicin, aclarubicin, procarbazine, cisplatin, methotrexate, benzylpenicillin, amoxicillin, penicillin, oxycillin, methicillin, carbenicillin, ampicillin, cefalexin, cefazolin, erythromycin, kitasamycin, chloramphenicol, thiamphenicol, minocycline, lincomycin, clindamycin, streptomycin, kanamycin, fradiomycin, gentamycin, spectinomycin, neomycin, vanomycin, tetracycline, ciprofloxacin, sulfanilic acid, cycloserine, sulfisomidine, isoniazid, ethambutol, acyclovir, gancyclovir, vidabarine, azidothymidine, dideoxyinosine, dideoxycytosine, morphine, codeine, oxycodone, hydrocodone, cocaine, pethidine, fentanyl, polymeric forms of any of the above drugs and any combinations thereof.

The water-soluble drug may not need to be converted to a salt form, i.e., tetracycline hydrochloride. In some embodiments, the therapeutic agent may include an anesthetic, i.e., bupivicaine, lidocaine, benzocaine, and the like.

Although the above therapeutic agents have been provided for the purposes of illustration, it should be understood that the present disclosure is not so limited. In particular, although certain therapeutic agents are specifically referred to above, the present disclosure should be understood to include analogues, derivatives and conjugates of such agents.

In should be noted that the therapeutic agent(s) may be combined with any portion of the medical device, including the mesh and/or the film layer. In some embodiments, the therapeutic agent may be included in the film to provide sustained release of the therapeutic agent following implantation. In some embodiments, the therapeutic agent may be included in the mesh.

In still other embodiments, the therapeutic agent(s) may be positioned with the perforations defined within the film. In such an embodiment, release of the therapeutic agent(s) may be prompted by the separation of a first portion of the film from a second portion of the film via the tearing along the perforations.

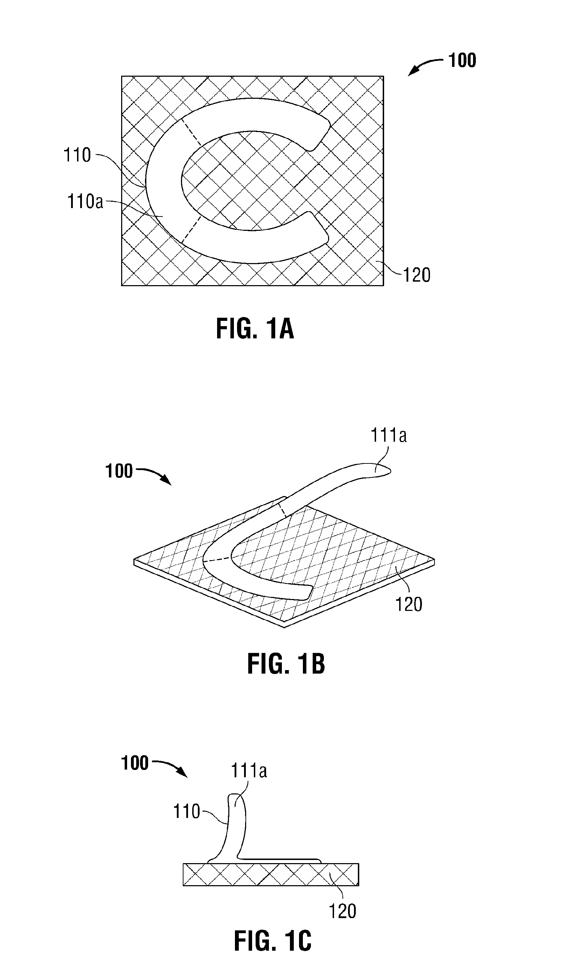

Turning now to FIG. 1A, implantable medical device 100 is illustrated including perforated film 110 positioned on mesh 120 wherein film 110 covers a portion of at least one side of mesh 120. Fixed portion 110a is attached to mesh 120 by any suitable means in the art and as described herein. Although film 110 is shown in contact with mesh 120, it is envisioned that film 110 has the ability to cover mesh 120 partially. In addition, although film 110 is shown having a "u" shape, it is envisioned that film 110 may be formed into any suitable shape and as mentioned herein.

Referring to FIGS. 1B and 1C, implantable medical device 100 is shown including film 110 having at least one perforated portion wherein finger 111a can be configured and dimensioned to be extended upward into a vertical or substantially perpendicular configuration relative to mesh 120. Because finger 111a is not fixedly attached to mesh 120, finger 111a of film 110 may be manipulated away from mesh 120, while fixedly attached portion 110a of film 110 remains in contact with mesh 120. Fingers 111a may be positioned along wound area or incision space to allow for direct treatment of tissue. For example, during a hernia wound repair, mesh 120 may be placed directly at or near the incision point so that fingers 111a may be positioned perpendicularly to the mesh 120 (FIG. 1C) and directly on each side of an incision to provide therapeutic agents to the wound (not shown). In alternative embodiments, fingers 111a may be used to wrap around sensitive tissue and/or organs.

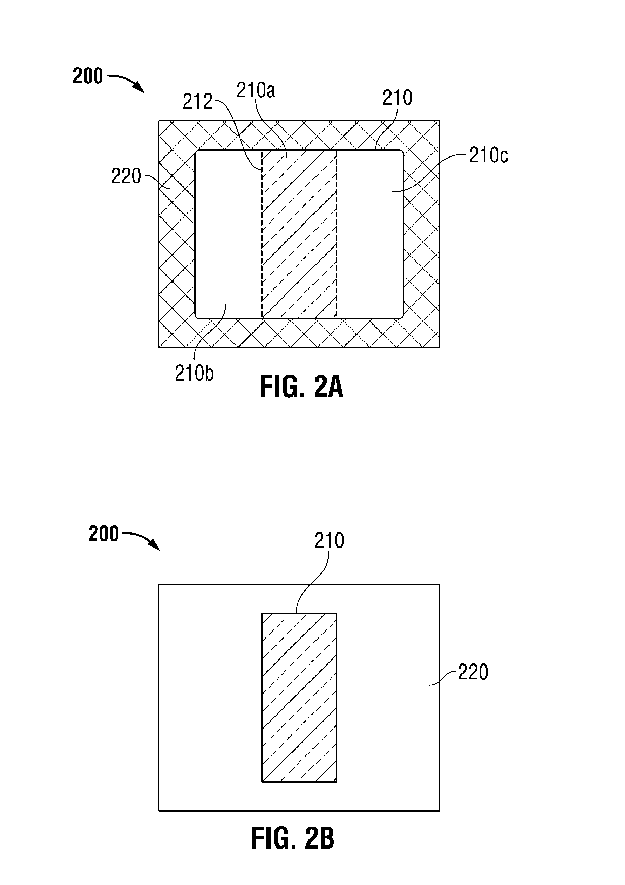

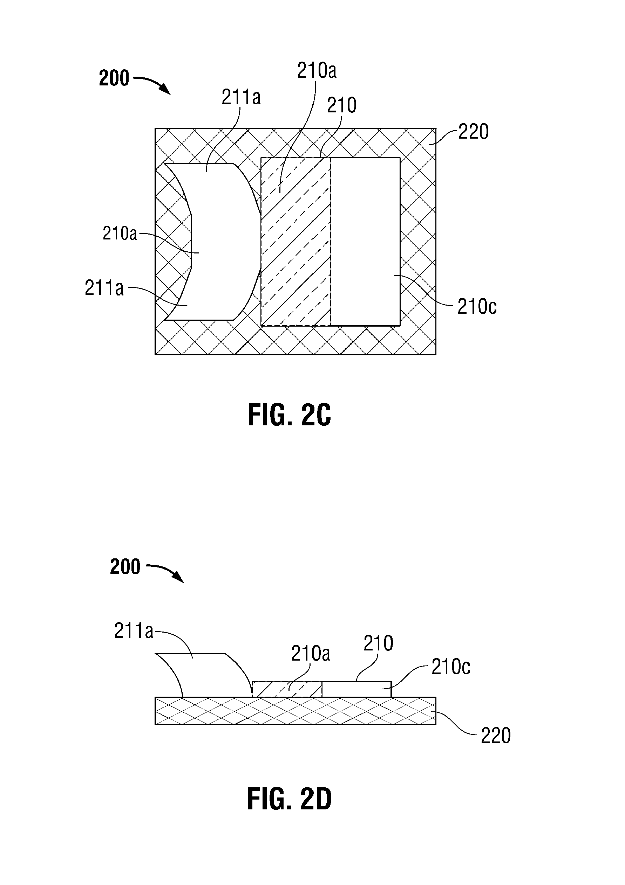

Referring to FIGS. 2A, 2B and 2C, an implantable medical device 200 is illustrated having a mesh 220 and a rectangular perforated film 210 thereon. Film 210 includes three sections separated by perforations 212, two outer sections 210b and 210c which are positionable away from mesh 220 and a middle section 210a which is fixedly attached to mesh 220. Although middle section 210a is shown in contact with mesh 220, it is envisioned that film section 210a has the ability to at least partially cover mesh 220. It is also envisioned that middle section 210a may not be fixedly attached to mesh 220 and at least one of the outer sections 210b and 210c or both outer sections 210b and 210c may be fixedly attached to the mesh. Suitable methods of attachment are described herein. In addition, although film 210 is shown having a rectangular shape, it is envisioned that film 210 may be formed into other shapes as mentioned above.

In embodiments, implantable medical device 200 may include film 210 having perforations 212 which may extend completely across film 210 (FIG. 2A). In such embodiments, at least one of outer sections 210b and 210c may be completely separated from implant 200 via tearing along perforations 212 (FIG. 2B). It is envisioned that the ability to completely separate portions of the film may be useful in controlling drug delivery in embodiments wherein the film includes at least one therapeutic agent. It is further envisioned that following separation, a user may reposition the separated film portion(s) to another portion of the mesh and/or to a different area of the wound site, i.e., the opposite side of the wound, a separate or different tissue layer, etc. Unlike prior art implants, the perforated films ability to separate from the mesh during a surgical procedure provides a user with the ability to change the implant as dictated by the actual condition of the wound site and/or the physical condition of the patient.

In alternative embodiments, perforations 212 may not extend completely across the face of film 210 (FIG. 2C) such that fingers 211a are formed and can be extended upward into a vertical or substantially perpendicular configuration and free of mesh 220 (FIG. 2D). Fingers 211a may be positioned across a wound area or incision space to allow for direct treatment of tissue.

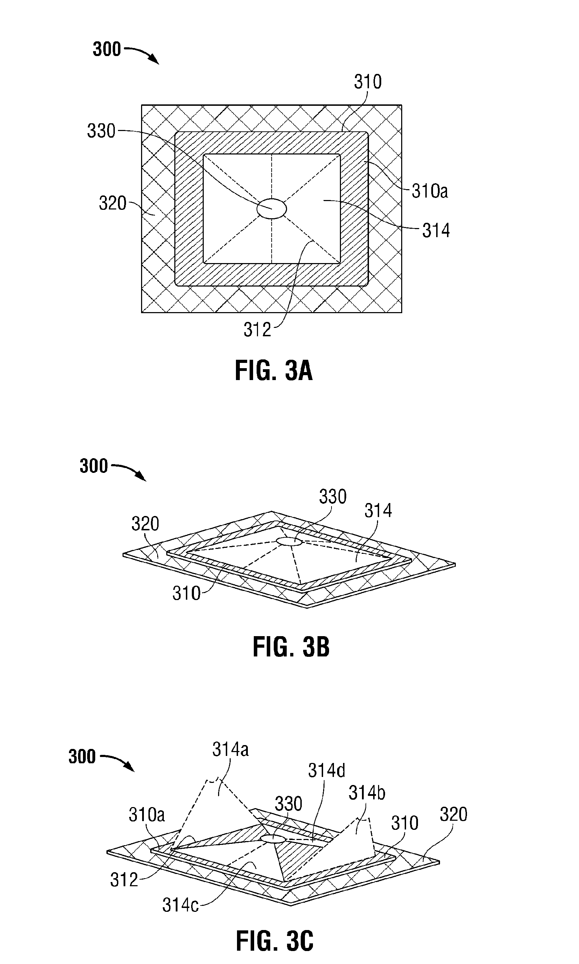

Turning now to FIG. 3A, implantable medical device 300 is illustrated including film 310 positioned on mesh 320 wherein film 310 is fixedly attached to the mesh 320 on the film's 310 outer periphery or edge portion 310a. A plurality of perforations 312 are shown extending from pre-cut hole or notch 330 such that a surgeon may manipulate or extend outward perforated portions 314 individually or collectively so that direct treatment of tissue surrounding the mesh 320 is allowed. Although notch 330 is shown positioned central to the film, the notch may be disposed in other positions relative to the film. Although film 310 is shown fixedly attached to mesh 320 via the outer periphery or edge portion 310a, it is envisioned that film 310 can be attached to mesh 320 via any other portion of the film 310. In addition, although film 310 is shown having a rectangular shape, it is envisioned that film 310 may be formed into other shapes as mentioned above.

In embodiments, film 310 may be formed into a tent-like arrangement as illustrated in FIG. 3B. In embodiments, film 310 may be manipulated to extend outward as shown in FIGS. 3C and 3D such that perforated portions 314a-d, alternatively or as a whole, may be extended upward into a vertical or substantially perpendicular configuration and free of or spaced a distance from mesh 320. Perforated portions 314a and/or 314b and/or perforated portions 314c and/ or 314d may extend upward and over the mesh area onto the wound site or patient tissue. It is envisioned that a surgeon may use such an arrangement to create space between the perforated portion of the film and the mesh which may be utilized for a variety of potential reasons. For instance, the surgeon may insert at least one finger or surgical tool through hole 330 and into the space between the film and the mesh to ensure the mesh is completely flat or unrolled. In another example, a surgical tool, such as a stapler, clip applier, suturing device, ultrasonic sprayer and the like, may be positioned through the hole and into the space between the film and the mesh to secure the mesh to tissue.

As mentioned above, and shown in Fig. 3C, first perforated portions 314a and 314b may be moved away from mesh 320 while second perforated portions 314c and 314d remain positioned near mesh 320. It is further envisioned that first perforated portions 314a and 314b may be positioned across a wound area or incision space to allow for direct treatment of tissue while second perforated portions 314c and 314d remain between the wound tissue and the mesh. Although film 310 is shown with a plurality of perforations extending outward from the center of the film, it is envisioned that film 310 may be cut into predetermined shapes using any known tool in the art, such as a scalpel or grasper such that perforated portions 314a and 314b may be positioned away from mesh 320.

The implants described herein may be useful in many endoscopic, laparoscopic, arthroscopic, endoluminal, transluminal, and/or open surgical procedures. Some examples include hernia repair, repair of vaginal prolapse, ligament repair, tendon repair, and the like. Although the polymeric films described herein may be made from any biocompatible materials, in certain procedures, the film layers may be made from anti-adhesive materials. For example, when implanting the medical devices described herein into hard to reach tissue, it might be useful to manipulate the implantable device 600 into a rolled configuration as shown in FIG. 4. Rolled implantable medical device 600 may be inserted through the elongated tubular member of a surgical device such as a laparoscope, endoscope, trocar, cannula, obturator, and the like, and subsequently placed on hard to reach tissue areas. In embodiments, implantable medical device 600 may include a perforated film 610 to prevent mesh 620 from becoming entangled to itself in the rolled configuration. After being unrolled at the site of implantation, portions of the perforated film 610 may be removed or trimmed as needed and/or may be implanted elsewhere in the surgical field.

Methods of delivery of a therapeutic agent are also provided herein which includes implanting a medical device including a mesh, and a film positioned on at least a portion of the mesh, wherein the film includes at least one perforation; optionally trimming at least one perforated portion of the film to a predetermined configuration; and positioning the trimmed perforated portion away from the mesh and onto a wound site. In certain embodiments, as mentioned above, the methods further include rolling the medical device; inserting the rolled medical device through an instrument having an elongated tubular member; and positioning the medical device at or near the wound site.

It will be understood that various modifications may be made to the embodiments disclosed herein. For example, in embodiments the medical device may be rolled prior to being delivered into the body via a cannula, trocar or laparoscopic delivery device. In another example, the medical devices described herein may be sterilized and packaged into using any suitable sterilization process, i.e., gamma radiation, and any suitable medical device package, i.e., an injectable medical device package. In still other examples, the implants described herein may include more than one film, mesh, and/or perforation. Thus, those skilled in the art will envision other modifications within the scope and spirit of the claims.

The invention may be described by reference to the following numbered paragraphs:-
1. An implantable medical device comprising:
   a mesh, and
   a perforated film positioned on at least a portion of the mesh.
2. The implantable medical device according to paragraph 1, wherein the film includes at least one perforation configured and dimensioned such that at least one perforated portion of the film is positionable away from the mesh.
3. The implantable medical device of paragraph 1, wherein the at least one perforated portion of the film is positioned perpendicularly away from the mesh.
4. The implantable medical device of paragraph 1, wherein the at least one perforated portion of the film is trimmable to a predetermined shape and size at the surgical site.
5. The implantable medical device of paragraph 1, wherein the at least one perforated portion of the film is trimmable to a predetermined shape and size prior to implantation.
6. The implantable medical device of paragraph 1, wherein the film includes graduated markings.
7. The implantable medical device of paragraph 1, wherein the film comprises an area larger than the mesh.
8. The implantable medical device of paragraph 1, wherein the film comprises an area smaller than the mesh.
9. The implantable medical device of paragraph 1, wherein the film further comprises at least one therapeutic agent.
10. The implantable medical device of paragraph 1, wherein the film is attached to the mesh on at least one peripheral edge of the film.
11. The implantable medical device of paragraph 1, wherein the film is attached to the mesh on at least one interior edge of the film.
12. The implantable medical device of paragraph 1, wherein the film is attached to the mesh on at least an interior portion of the mesh.
13. The implantable medical device of paragraph 1, wherein the film is attached to the mesh on both interior and exterior portions of the film to form a tent-like attachment configuration.
14. The implantable medical device of paragraph 1, wherein the film comprises pre-cut holes.
15. The implantable medical device of paragraph 1, wherein the mesh further comprises at least one grip member.
16. The implantable medical device of paragraph 1, wherein at least a portion of the film is fixedly attached to the mesh.
17. An implantable medical device comprising:
   a mesh; and
   a film with a plurality of perforations configured and dimensioned such that at least one portion of the film is fixedly attached to the mesh and at least one portion of the film is positionable away from the mesh.
18. A method of delivery of a therapeutic agent comprising:
   implanting a medical device including:
      a therapeutic agent,
      a mesh, and
      a film positioned on at least a portion of the mesh, wherein the film includes at least one perforated portion; and
   positioning the perforated portion away from the mesh and onto a wound site.
19. The method according to paragraph 18, further comprising:
   trimming the at least one perforated portion of the film to a predetermined configuration.
20. The method according to paragraph 18, further comprising:
   rolling the medical device;
   inserting the rolled medical device through an instrument having an elongated tubular member; and
   positioning the medical device at or near the wound site.

## Claims

1. An implantable medical device comprising:
a mesh, and
a perforated film positioned on at least a portion of the mesh.

2. The implantable medical device according to claim 1, wherein the film includes at least one perforation configured and dimensioned such that at least one perforated portion of the film is positionable away from the mesh.

3. The implantable medical device of claim 1 or claim 2, wherein the at least one perforated portion of the film is positioned perpendicularly away from the mesh.

4. The implantable medical device of any preceding claim, wherein the at least one perforated portion of the film is trimmable to a predetermined shape and size at the surgical site; and/or wherein the at least one perforated portion of the film is trimmable to a predetermined shape and size prior to implantation.

5. The implantable medical device of any preceding claim, wherein the film includes graduated markings.

6. The implantable medical device of any preceding claim, wherein the film comprises an area larger than the mesh, or wherein the film comprises an area smaller than the mesh.

7. The implantable medical device of any preceding claim, wherein the film further comprises at least one therapeutic agent.

8. The implantable medical device of any preceding claim, wherein the film is attached to the mesh on at least one peripheral edge of the film; and/or wherein the film is attached to the mesh on at least one interior edge of the film.

9. The implantable medical device of any preceding claim, wherein the film is attached to the mesh on at least an interior portion of the mesh.

10. The implantable medical device of any preceding claim, wherein the film is attached to the mesh on both interior and exterior portions of the film to form a tent-like attachment configuration.

11. The implantable medical device of any preceding claim, wherein the film comprises pre-cut holes.

12. The implantable medical device of any preceding claim, wherein the mesh further comprises at least one grip member.

13. The implantable medical device of any preceding claim, wherein at least a portion of the film is fixedly attached to the mesh.

14. An implantable medical device comprising:
a mesh; and
a film with a plurality of perforations configured and dimensioned such that at least one portion of the film is fixedly attached to the mesh and at least one portion of the film is positionable away from the mesh.
